# EUROPÄISCHE PATENTANMELDUNG

(11) **EP 2 168 485 A1**
(43) Veröffentlichungstag der Anmeldung: **31.03.2010**
(21) Anmeldenummer: 09154854.5
(22) Anmeldetag: 11.03.2009
(51) Int. Cl.: A61B 6/00, A61B 6/03, A61B 6/04

(54) **Brustfixierung für ein Untersuchungsgerät zur Untersuchung der weiblichen Brust**

(30) Priorität: 29.09.2008 DE 102008042430
(71) Anmelder: MIR Medical Imaging Research Holding GmbH, 91096 Möhrendorf (DE)
(72) Erfinder: Schilling, Harry, 85072, Eichstätt (DE); Kalender, Willi, 91096, Möhrendorf (DE)
(74) Vertreter: Lohr, Georg

(57) **Zusammenfassung**

Eine erfindungsgemäße Vorrichtung zur Fixierung einer weiblichen Brust umfasst eine Fixiervorrichtung, welche in eine Patientenliege einsetzbar ist. Durch unterschiedliche Größen und Formen der Fixiervorrichtung ist diese an unterschiedliche Brustformen anpassbar. Zur Identifikation der Fixiervorrichtung (40) weist die Fixiervorrichtung einen RFID-Transponder (90) auf. Damit kann mit der Bilddatenaufzeichnung auch die verwendete Fixiervorrichtung automatisch erfasst werden. Damit kann bei der Bildauswertung die Form der Fixiervorrichtung berücksichtigt werden. Weiterhin kann sichergestellt werden, dass bei späteren Aufnahmen wieder dieselbe Form der Fixiervorrichtung verwendet wird, so dass die Aufnahmen vergleichbar bleiben.

## Beschreibung

### Technisches Gebiet

Die Erfindung betrifft eine Vorrichtung zur Fixierung der Brust einer Patientin in einem Untersuchungsgerät zur Untersuchung der weiblichen Brust. Ein solches Untersuchungsgerät kann ein Röntgengerät zur Abbildung der weiblichen Brust (Mammografie), ein CT-Scanner oder auch ein Ultraschallgerät sein. Weiterhin betrifft die Erfindung ein Röntgengerät beziehungsweise einen CT Scanner mit einer entsprechenden Fixierung der Brust einer Patientin sowie ein Verfahren zum Betrieb eines solchen Gerätes.

### Stand der Technik

Zur Untersuchung der weiblichen Brust sind verschiedene Geräte, wie Röntgengeräte oder auch CT-Scanner bekannt. Ein solcher CT-Scanner ist beispielsweise in der US 2006/0094950 A1 offenbart. Unter einer Liege, auf der eine zu untersuchende Patientin liegt, befindet sich eine Röntgenvorrichtung mit einer rotierenden Gantry, welche eine Röntgenröhre und einen Detektor aufweist. Die zu untersuchende Brust der Patientin ragt durch eine Öffnung in der Liege in den Strahlengang der Röntgenvorrichtung. Um nun während der Untersuchung konstante Verhältnisse zu erzeugen, wird die zu untersuchende Brust mit einem Stempel nach oben gedrückt und in eine vordefinierte Form gebracht. Durch eine Verschiebung des Stempels ist eine Anpassung an unterschiedliche Brustgrößen möglich. Die Anpassung kann hier allerdings nur in der Länge und nicht im Durchmesser erfolgen. Eine andere Vorrichtung zur Stabilisierung der Brust der Patientin ist in der US 6,418,188 B1 offenbart. Ein Becher aus gummiartigem Gewebe wird über die Brust gestülpt und mittels einer Schnur von der Patientin weggezogen. Dadurch wird die Brust im Durchmesser komprimiert und in die Länge gezogen. Mit dieser Vorrichtung ist keine exakt reproduzierbare Lage und Form der Brust herstellbar. Eine verbesserte Vorrichtung ist in der US 2004/0082856 A1 offenbart. Austauschbare Einsätze in die Patientenliege sorgen durch ihre Außenkontur für eine feste Fixierung der Brust. Hier bleibt weiterhin die Problematik bestehen, dass bei wiederholten Untersuchungen unterschiedliche Einsätze mit unterschiedlichen Größen eingesetzt werden und dadurch die einzelnen Aufnahmen kaum mehr miteinander vergleichbar sind.

### Darstellung der Erfindung

Der Erfindung liegt die Aufgabe zugrunde, eine Vorrichtung zur Fixierung der Brust einer Patientin in einem Untersuchungsgerät zur Untersuchung der weiblichen Brust derart auszugestalten, dass die Brust der Patientin bei größtmöglichen Komfort für die Patientin in einer auch über mehrere Aufnahmen reproduzierbaren Lage fixiert werden kann. Ein weiterer Aspekt der Erfindung ist die Ausgestaltung eines Untersuchungsgerätes zur Untersuchung der weiblichen Brust, insbesondere eines Röntgengerätes beziehungsweise eines CT-Scanners mit einer erfindungsgemäßen Vorrichtung zur Fixierung der Brust. Schließlich ist noch ein Verfahren zum Betrieb eines solchen Gerätes Gegenstand der Erfindung.

Diese Aufgabe wird durch Vorrichtungen nach den unabhängigen Ansprüchen gelöst. Vorteilhafte Ausgestaltungen der Erfindung sind in den Unteransprüchen angegeben.

Eine erfindungsgemäße Vorrichtung zur Fixierung einer weiblichen Brust einer Patientin in einem Untersuchungsgerät umfasst einen Becher 40, welcher in eine Auflagefläche 20, die auch eine Patientenliege sein kann, einsetzbar ist. Zur Identifikation der Fixiervorrichtung weist die Fixiervorrichtung einen RFID-Transponder auf.

Es ist besonders vorteilhaft, wenn verschiedene Exemplare der Fixiervorrichtung in unterschiedlichen Größen und in unterschiedlichen, an verschiedene Brustformen angepassten Formen ausgeführt sind. Somit kann jeweils eine Fixiervorrichtung passend für die Brustform ausgewählt werden. Durch den integrierten RFID-Transponder kann sichergestellt werden, dass auch bei wiederholten Untersuchungen immer dieselbe Form beziehungsweise Größe der Fixiervorrichtung verwendet wird. Weiterhin kann bei der Auswertung der Bilddaten automatisch die Form der Fixiervorrichtung berücksichtigt werden. So kann die Kollimierung beziehungsweise das Messfeld aufgrund der Daten aus dem RFID-Transponder optimal eingestellt werden, um die Strahlenbelastung zu minimieren und die Bildqualität zu optimieren. Ebenso können auch noch weitere Parameter für Korrekturverfahren eingestellt werden Es kann insbesondere eine Seriennummer oder eine andere Kennzeichnung der Fixiervorrichtung automatisch ausgelesen und mit den Bilddaten aufgezeichnet werden. Grundsätzlich können auch andere Identifikationssysteme, wie beispielsweise Schaltnocken oder auch Barcodes vorgesehen sein. Weiterhin können noch Sensoren zur Erfassung biologischer, chemischer oder physikalischer Größen wie der Temperatur, des Druckes etc. an der Vorrichtung angebracht sein. Diese Sensoren können vorteilhafterweise per Funk oder über den RFID-Transponder abgefragt werden.

In einer weiteren vorteilhaften Ausgestaltung der Erfindung sind Verstärkungsrippen 44 vorgesehen, welche die Kontur einer Wandung 46 vorgegeben und diese gleichzeitig stabilisieren. Die Wandung selbst ist vorzugsweise aus einem durchstechbaren Material, so dass beispielsweise Kontrastmittel in die Brust injiziert oder Gewebeproben aus dieser entnommen werden können.

In einer anderen Ausgestaltung der Erfindung ist der Becher 40 aus einem optisch transparenten Material oder weist zumindest transparente Bereiche auf. Zusätzlich beziehungsweise alternativ können auch Markierungen an dem Becher 40 vorgesehen sein. Solche Markierungen können beispielsweise Einstichpositionen für Biopsienadeln angeben, mit denen bestimmte Positionen im Inneren der Brust erreicht werden können. Weiterhin könnten auch Löcher beziehungsweise Öffnungen für medizinische Instrumente in dem Becher 40 vorgesehen sein.

Besonders vorteilhaft ist es, wenn ein Vakuumssystem vorgesehen ist, um die Brust exakt in die Form der Fixiervorrichtung zu bringen. Gegebenenfalls kann die Fixiervorrichtung auch durch das Vakuumssystem an der Brust gehalten werden. Für das Vakuumssystem ist ein Schlauchanschluss 43 oder ein anderes Mittel, wie beispielsweise eine Steckverbindung zum Anschluss einer Vakuumpumpe vorgesehen. Durch Absaugen der Luft im Innenraum der Fixiervorrichtung mittels Saugkanälen 45 passt sich die Brust exakt an die Form der Fixiervorrichtung an und die Fixiervorrichtung hält zudem fest an der Brust. Besonders günstig ist es, eine Vielzahl von Saugkanälen 45 und Luftaustrittsöffnungen, durch die Luft vom inneren der Fixiervorrichtung in die Saugkanäle austreten kann, vorzusehen.

Ein erfindungsgemäßer Satz Fixiervorrichtungen und umfasst mehrere der oben dargestellten Fixiervorrichtungen in unterschiedlichen Größen beziehungsweise mit Bechern unterschiedlicher Größen. Die Durchmesser der Becher liegen bevorzugt in einem Bereich von 80mm bis 180mmm. Weiterhin sind in dem RFID-Transponder einer jeder Fixiervorrichtung Informationen über deren Größe abgespeichert. Diese Informationen können unmittelbare Größenangaben, wie Durchmesser oder Länge sein. Sie können aber auch mittelbare Angaben, wie beispielsweise eine Seriennummer sein, anhand derer ein Lesegerät beispielsweise über in einer Datenbank abgespeicherte Informationen auf die Größe zurück schließen kann.

Ein weiterer Aspekt der Erfindung ist ein Röntgengerät, insbesondere einen CT-Scanner mit einer oben dargestellten Vorrichtung zur Brustfixierung. Weiterhin weist ein solches erfindungsgemäßes Röntgengerät ein Lesegerät für einen RFID-Transponder auf.

Besonders vorteilhaft ist es, wenn ein solches Röntgengerät eine Datenbank mit Scanparametern wie Spannung, Strom, Blende, Spirallänge, Wasserkorrekturparameter, Streustrahlenkorrekturparameter, Ringartefaktkorrekturparameter für verschiedene Vorrichtungen zur Fixierung der Brust aufweist oder zumindest eine Datenverbindung zu einer solchen Datenbank hat. Zudem ist es vorteilhaft, wenn in dieser Datenbank die entsprechenden RFID-Transponderkennungen enthalten sind.

Grundsätzlich ist es möglich, die Patientin auf der Auflagefläche 20 mit bereits eingesetztem Becher 40 zu platzieren. Besonders vorteilhaft ist es jedoch, wenn die Brustfixierung beziehungsweise der Becher vor der Platzierung der Patientin an der Auflagefläche bereits an der Patientin angelegt wird. Ein entsprechendes Verfahren umfasst die folgenden Schritte:
a. Anlegen des Bechers an der Brust sowie Aktivieren des Vakuums, und
b. Platzieren der Patientin an der Auflagefläche.

Hierzu wird vorzugsweise der Vakuumsschlauch durch den Brustausschnitt 21 der Auflagefläche gezogen.

Ein alternatives Verfahren umfasst die folgenden Schritte:
a. Halten des Bechers an der Brust, und
b. Platzieren der Patientin an der Auflagefläche.

Hierzu ist eine Halterung für den Becher an der Auflagefläche notwendig, welche sich selbsttätig arretiert, beispielsweise ein Magnetverschluss. Weiterhin wird bevorzugt automatisch, beispielsweise durch eine Magnetkupplung, die Vakuumverbindung hergestellt. Hierzu wird der Becher mit der Vakuumpumpe verbunden.

Ein Verfahren zur Verwendung eines erfindungsgemäßen Röntgengerätes, wie zuvor beschrieben, umfasst die folgenden Schritte:
a. Einsetzen der Fixiervorrichtung in eine Auflagefläche;
b. Auslesen des RFID-Transponders;
c. Überprüfen, ob die Fixiervorrichtung bereits verwendet wurde anhand der aus dem RFID-Transponder ausgelesenen Daten;
d. Ausgabe einer Fehlermeldung, wenn die Fixiervorrichtung bereits verwendet wurde. Optional kann das Verfahren an dieser Stelle abgebrochen werden.
e. Schreiben einer Markierung in den RFID-Transponder, dass die Fixiervorrichtung bereits verwendet wurde. Optional kann auch hier die Anzahl der Verwendungen in den RFID-Transponder geschrieben werden, oder es kann auch ein entsprechender Zähler in dem RFID-Transponder erhöht werden.
f. Automatische Auswahl wenigstens eines optimalen Scanparameters aus Spannung, Strom, Blende, Spirallänge, Wasserkorrekturparameter, Streustrahlenkorrekturparameter, Ringartefaktkorrekturparameter aufgrund der aus dem RFID-Transponder ausgelesenen Daten.
g. Durchführen des Scans, d. h. der Röntgenabbildung.

Zur Auswahl der Daten in Schritt f. ist vorzugsweise eine Datenbank oder zumindest eine Tabelle mit geeigneten Parametern für verschiedene RFID-Transponder-Kennungen in dem Röntgengerät vorhanden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
- Figur 1: zeigt eine Erfindungsgemäße Vorrichtung zur Fixierung einer weiblichen Brust.
- Figur 2: zeigt eine weitere Vorrichtung zur Fixierung einer weiblichen Brust mit einer Gitterstruktur.
- Figur 3: zeigt eine weitere Vorrichtung zur Fixierung einer weiblichen Brust.
- Figur 4: zeigt eine kleinere Vorrichtung zur Fixierung einer weiblichen Brust.
- Figur 5: zeigt die Integration einer erfindungsgemäßen Vorrichtung.
- Figur 6: zeigt ein Röntgengerät zur Abbildung einer weiblichen Brust.
- Figur 7: zeigt eine erfindungsgemäße Vorrichtung zur Brustfixierung, welche in eine Patientenliege eines Röntgengerätes integriert ist.
- Figur 8: zeigt ein vertikal angeordnetes Röntgengerät.

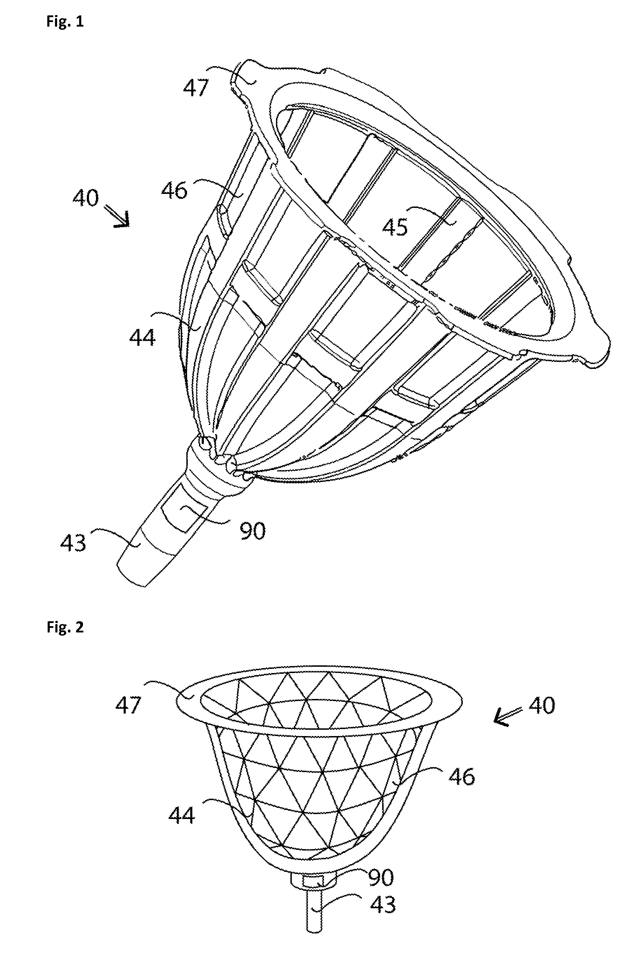

Figur 1 zeigt eine erfindungsgemäße Vorrichtung zur Fixierung einer weiblichen Brust. Die Fixiervorrichtung weist einen Becher 40 zur Aufnahme der Brust auf. Die Grundform der Wandung 46 des Bechers 40 wird durch Verstärkungsrippen 44 vorgegeben und stabilisiert. Der Becher 40 kann neben einer wie in der Figur 1 dargestellten, der Brust angepassten Form auch eine zylindrische, eine kegelförmige, eine halbkugelförmige oder jede andere adäquate Form aufweisen. Die Wandung selbst ist vorzugsweise aus einem durchstechbaren Material, so dass beispielsweise Kontrastmittel in die Brust injiziert oder Gewebeproben aus dieser entnommen werden können. An dem oberen Ende des Bechers 40, entsprechend dem oberen Ende der Vorrichtung, welches in Richtung der Brustwand der Patientin zeigt, befindet sich eine Öffnung, die hinreichend groß ist, um die Brust der Patientin zu umschließen. Diese Öffnung ist von einem Abschlussrand 47 umschlossen. Er erhöht weiterhin die Stabilität und weist Laschen auf, mit denen dieser an der Auflagefläche 20 befestigt werden kann. Entsprechend der Brustgröße der Patientin sind verschiedene Größen und Formen der Becher 40 vorgesehen. Um die Brust nun exakt in die Form der Becher 40 zu bringen, weist die Fixiervorrichtung ein Vakuumssystem auf. Durch mindestens einen Saugkanal 45, der bevorzugt in eine Verstärkungsrippe 44 integriert ist, wird die Luft aus dem Innenraum des Bechers abgesaugt. Hierzu ist ein Schlauchanschluss 43 zum Anschluss einer Vakuumpumpe vorgesehen. Durch Absaugen der Luft im Innenraum passt sich die Brust exakt an die Form des Bechers der Fixiervorrichtung an und die Fixiervorrichtung hält zudem fest an der Brust. Besonders günstig ist es, eine Vielzahl von Saugkanälen und entsprechenden Öffnungen vorzusehen. Zur Identifikation des Bechers ist ein RFID-Transponder (90) vorgesehen. In diesem Transponder können technische Informationen zur Fixiervorrichtung beziehungsweise zum Bechers selbst, wie beispielsweise über Größe, Material, Sterilität, Röntgeneigenschaften, Herstellungsdatum, Nutzungsdauer, Anzahl der Einsätze beziehungsweise durchgeführte Untersuchungen, Seriennummer, Eignung zum Einsatz in einem bestimmten Röntgengerät etc. gespeichert sein. Diese Daten können einerseits bei der Herstellung in den Transponder programmiert oder auch während des Einsatzes programmiert oder aktualisiert werden.

Figur 2 zeigt eine weitere Ausgestaltung einer erfindungsgemäßen Vorrichtung zur Brustfixierung. Hier umfasst die Wandung 46 des Bechers 40 ein Gewebegitter. Dieses Gewebegitter kann auch so gestaltet sein, dass es sich bei Zug zusammenzieht und die Brust komprimiert. In diesem Falle müsste eine Zugkraft an dem Ende auf Seiten des Schlauchanschlusses 43 aufgebracht werden. Es kann ein engmaschiges Gewebegitter selbst zur Brustfixierung verwendet werden. Allerdings ist hier das Unterdrucksystem noch nicht einsetzbar, da zwischen den Maschen des Gewebes wieder Luft eindringen würde. Wird nun die Vorrichtung auf der Innenseite mit einer elastischen Folie oder Beschichtung versehen, so kann sie dadurch luftdicht gemacht werden, so dass wieder die Luft aus dem Zwischenraum zwischen der Wandung 46 und der Brust abgesaugt werden kann.

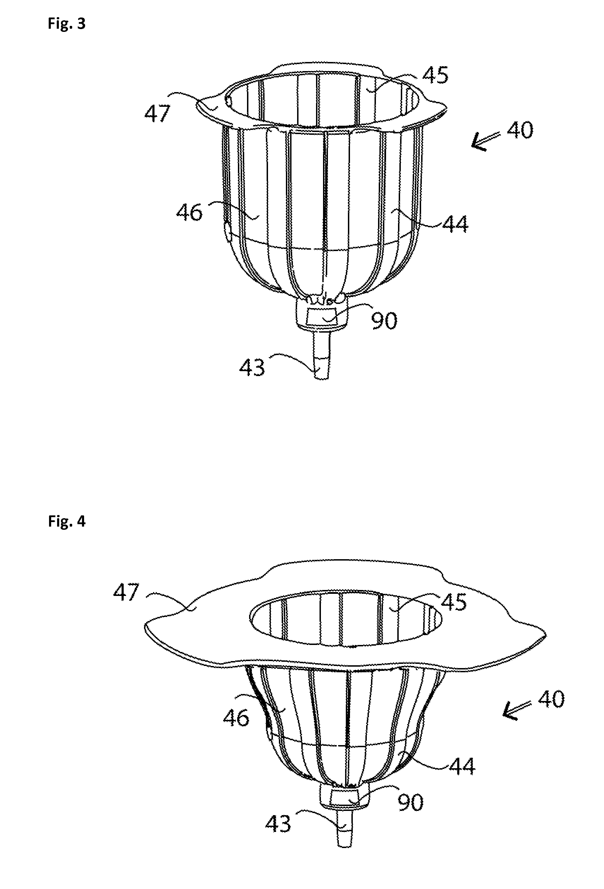

In Figur 3 ist eine weitere Ausgestaltung einer erfindungsgemäßen Vorrichtung zur Brustfixierung angegeben. Der Becher der Fixiervorrichtung basiert hier auf einer näherungsweise zylindrischen Grundform. Die übrigen Merkmale entsprechen der Figur 1.

In Figur 4 ist eine ähnliche Vorrichtung zur Brustfixierung wie in Figur 3 offenbart. Allerdings ist diese Vorrichtung für kleinere Brüste mit einer kleineren Körbchengröße ausgelegt. Sie kann alternativ zur Vorrichtung aus Figur 3 eingesetzt werden.

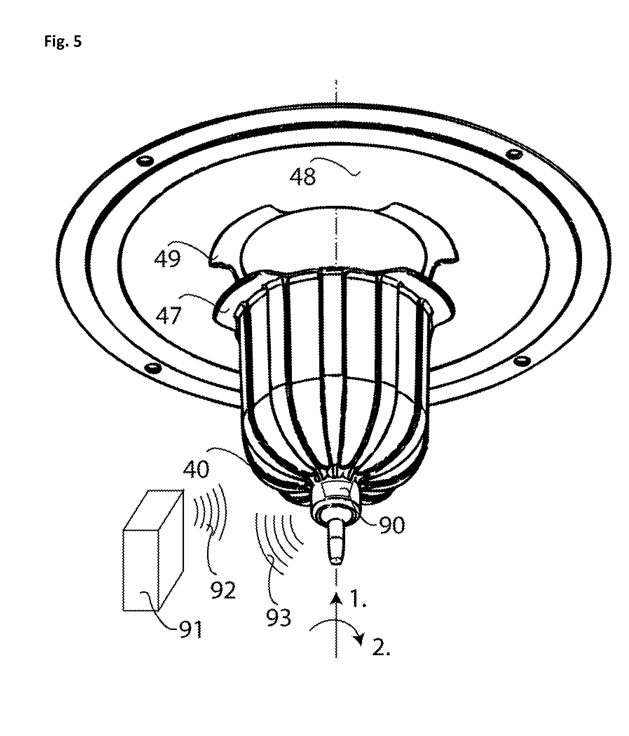

Figur 5 zeigt die Integration einer erfindungsgemäßen Vorrichtung zur Brustfixierung in eine Auflagefläche 20. Ein Aufnahmering 48 ist in die Auflagefläche integriert. Er kann auch zur groben Adaption an unterschiedliche Brustgrößen verwendet werden. Die Fixiervorrichtung wird hier beispielhaft mittels Laschen des Abschlussrandes 47 an dem Aufnahmering 48 befestigt. Dazu wird sie zuerst nach oben in die Führungsnut 49 geschoben (1.) und dann verdreht (2.). Das Prinzip entspricht dem eines Bajonettverschlusses. selbstverständlich sind auch andere Verschlussmechanismen, wie beispielsweise ein Magnetverschluss, ein Schnappverschluss oder auch ein Klettverschluss realisierbar. Wesentlich ist, dass die Fixiervorrichtung einfach austauschbar ist. Wie gezeigt, wird die Vorrichtung von unten, das heißt von der der Patientin abgewandten Seite der Auflagefläche vorgenommen. Dies erleichtert auch den Anschluss des Vakuumsschlauches. Besonders günstig ist es jedoch, wenn die Fixiervorrichtung von der Patientenseite aus eingesetzt werden kann, denn dann kann die der Patientin abgewandte Seite der Auflagefläche vollständig in ein geschlossenes Gehäuse integriert werden. Für diesen Fall wird vorteilhafterweise ein Stecksystem für den Vakuumanschluss zur Luftabsaugung vorgesehen. Ein solches Stecksystem ist auch mit einer Magnetkupplung besonders einfach realisierbar. In dieser Figur ist weiterhin noch die Kommunikation zwischen dem RFID-Transponder 90 und dem RFID-Lesegerät 91 schematisch dargestellt. Zunächst sendet das RFID-Lesegerät 91 ein Anfragesignal 92, welches dann von dem RFID-Transponder 90 mittels des Antwortsignals 93 beantwortet wird. Unter RFID (Radio Frequency Identification) wird hier eine Identifizierung durch Funksignale oder andere elektromagnetische Signale im Allgemeinen verstanden. Vorzugsweise wird aus Kostengründen ein passiver Transponder eingesetzt, der seine Energie durch das Lesegerät erhält. Besonders bevorzugt wird ein RFID-System entsprechend einer der folgenden Normen eingesetzt: ISO/IEC 10536, ISO/IEC 14443, ISO/IEC 15693, ISO 69873.

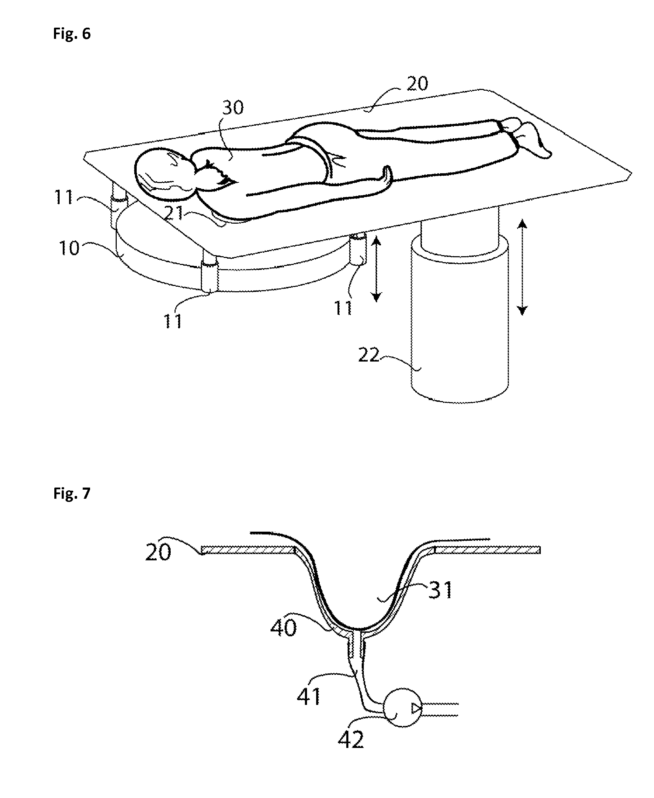

In Figur 6 ist ein Röntgengerät zur Abbildung einer weiblichen Brust dargestellt. Auf der Patientenliege 20 liegt eine Patientin 30. Die zu untersuchende Brust hängt über einen Brustausschnitt 21 durch die Patientenliege 20 in den Aufnahmebereich einer Gantry 10 und wird dort durch eine erfindungsgemäße Brustfixierung gehalten. Diese Brustfixierung ist aus dieser zeichnerischen Perspektive nicht erkennbar, das sie durch den Körper der Patientin abgedeckt wird. Die Gantry 10 ist eine Spiral-Computertomographen Gantry mit einer Röntgenröhre und einem Detektor, welche sich um die zu untersuchende Brust drehen. Während der Drehung wird die Brust abgebildet. Gleichzeitig mit der Drehung wird über den Gantryhubantrieb 11 eine Verschiebung in vertikaler Richtung durchgeführt, so dass die Brust spiralförmig abgetastet wird. Die Patientenliege 20 ist über einen Patientenliegenhubantrieb 22 in der Höhe verstellbar. Optional kann bei einem fest installierten Patiententisch dieser auch noch um die Achse des Patientenliegenhubantriebs 22 drehbar sein.

Figur 7 zeigt exemplarisch eine Fixiervorrichtung, bei der der Becher 40, die in die Auflagefläche 20 eingesetzt ist. Die Verbindung erfolgt hier beispielhaft über eine flache Kegelpassung. Über einen Schlauch 41 ist eine Vakuumpumpe 42 angeschlossen, so dass die Brust 31 der Patientin in der Fixiervorrichtung durch Unterdruck fixiert wird.

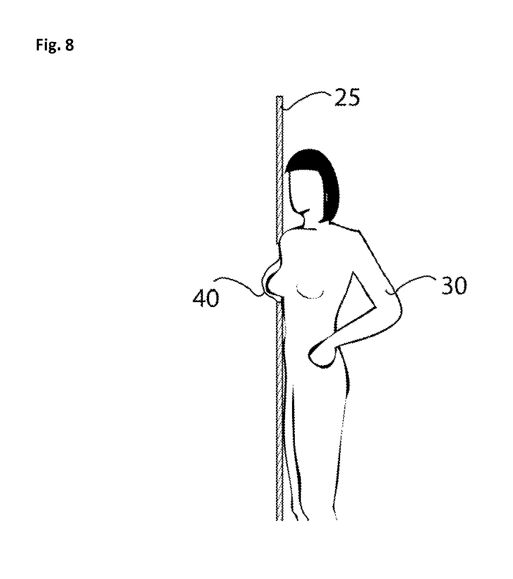

Figur 8 zeigt exemplarisch ein vertikal angeordnetes Röntgengerät, bei dem der Becher 40 in eine vertikale Auflagefläche eingesetzt ist.

### Bezugszeichenliste

- 10: Gantry
- 11: Gantryhubantrieb
- 20: Auflagefläche
- 21: Brustausschnitt
- 22: Patientenliegenhubantrieb
- 25: Trennwand
- 30: Patientin
- 31: Brust
- 40: Becher
- 41: Schlauch
- 42: Vakuumpumpe
- 43: Schlauchanschluss
- 44: Verstärkungsrippe
- 45: Saugkanal
- 46: Wandung
- 47: Abschlussrand
- 48: Aufnahmering
- 49: Führungsnut
- 90: RFID Transponder
- 91: RFID Lesegerät
- 92: RFID Anfragesignal
- 93: RFID Antwortsignal

## Patentansprüche

1. Vorrichtung zur Fixierung einer weiblichen Brust (31) einer Patientin (30) in einem Untersuchungsgerät, umfassend einen Becher (40) zur Aufnahme der Brust, welcher in eine Öffnung einer Auflagefläche (20) für die Patientin (30) eingebracht werden kann,
**dadurch gekennzeichnet, dass**
an dem Becher (40) ein RFID-Transponder (90) angebracht ist.

2. Vorrichtung nach Anspruch 1,
**dadurch gekennzeichnet, dass**
in dem ein RFID-Transponder (90) wenigstens eine der folgenden Informationen gespeichert ist: Größe und/oder Material des Bechers, Sterilität, Röntgeneigenschaften, Herstellungsdatum, Nutzungsdauer, Anzahl der durchgeführten Untersuchungen, Seriennummer.

3. Vorrichtung nach Anspruch 1 oder 2,
**dadurch gekennzeichnet, dass**
der Becher (40) eine Wandung (46), welche durch Verstärkungsrippen (44) verstärkt ist umfasst.

4. Vorrichtung nach einem der vorhergehenden Ansprüche,
**dadurch gekennzeichnet, dass**
an dem Becher (40) Mittel (43) zum Anschluss einer Vakuumpumpe vorgesehen sind.

5. Vorrichtung nach einem der vorhergehenden Ansprüche,
**dadurch gekennzeichnet, dass**
der Becher (40) ein optisch transparentes Material umfasst.

6. Vorrichtung nach einem der vorhergehenden Ansprüche,
**dadurch gekennzeichnet, dass**
der Becher (40) Markierungen für bestimmte Positionen im Inneren der Brust umfasst.

7. Vorrichtung nach einem der vorhergehenden Ansprüche,
**dadurch gekennzeichnet, dass**
der Becher (40) Öffnungen für medizinische Instrumente umfasst.

8. Vorrichtungssatz zur Fixierung einer weiblichen Brust einer Patientin (30) in einem Untersuchungsgerät, umfassend mehrere Vorrichtungen entsprechend einem der vorhergehenden Ansprüchen mit Bechern (40) in verschiedenen Größen und einem RFID-Transponder (90), in welchem jeweils eine Kennung zur Identifizierung der entsprechenden Größe der Becher gespeichert ist.

9. Röntgengerät zur Abbildung einer Brust einer Patientin (30), mit
- einer Röntgeneinrichtung umfassend eine Röntgenröhre und einen Röntgendetektor,
- einer Auflagefläche (20) zur Aufnahme einer Patientin (30) mit einem Brustausschnitt (21),
- wenigstens einer Vorrichtung zur Fixierung der Brust nach einem der vorhergehenden Ansprüche mit einem RFID-Transponder (90) und
- mit einem RFID-Lesegerät zur Abfrage von Daten des RFID-Transponders und/oder Speicherung von Daten in dem RFID-Transponder wenigstens einer der Vorrichtungen zur Fixierung der Brust.

10. Röntgengerät nach Anspruch 9,
**dadurch gekennzeichnet, dass**
das Röntgengerät eine Datenbank mit Scanparametern für verschiedene Vorrichtungen zur Fixierung der Brust aufweist oder zumindest eine Datenverbindung zu einer solchen Datenbank hat.

11. Röntgengerät zur Abbildung einer Brust einer Patientin (30), mit
- einer Röntgeneinrichtung umfassend eine Röntgenröhre und einen Röntgendetektor,
- einer Auflagefläche (20) zur Aufnahme einer Patientin (30) mit einem Brustausschnitt (21),
- wenigstens einer Vorrichtung zur Fixierung der Brust nach einem der vorhergehenden Ansprüche mit einem RFID-Transponder (90) und
- mit einem RFID-Lesegerät zur Abfrage von Daten des RFID-Transponders und/oder Speicherung von Daten in dem RFID-Transponder wenigstens einer der Vorrichtungen zur Fixierung der Brust.

12. Röntgengerät nach Anspruch 11, welches ein CT-Scanner ist.

13. Verfahren zum Anlegen einer Vorrichtung nach Anspruch 1 zur Fixierung einer weiblichen Brust (31) an eine Patientin (30) umfassend die Schritte:
a. Anlegen des Bechers (40) der Vorrichtung an der Brust (31) sowie Aktivieren eines Vakuums zum Ansaugen des Bechers an die Brust, und
b. Platzieren der Patientin an der Auflagefläche (20).

14. Verfahren zum Anlegen einer Vorrichtung nach Anspruch 1 zur Fixierung einer weiblichen Brust (31) an eine Patientin (30), umfassend die Schritte:
a. Halten des Bechers (40) der Vorrichtung an der Brust (31),
b. Platzieren der Patientin an der Auflagefläche, und
c. Herstellen einer Verbindung zwischen dem Becher (40) und einer Vakuumpumpe (42).

15. Verfahren zur Verwendung eines Röntgengerätes nach Anspruch 11, umfassend die Schritte:
a. Einsetzen der Fixierung der Brust in eine Auflagefläche;
b. Auslesen des RFID-Transponders;
c. Überprüfen, ob die Fixiervorrichtung bereits verwendet wurde anhand der aus dem RFID-Transponder ausgelesenen Daten;
d. Ausgabe einer Fehlermeldung, wenn die Fixiervorrichtung bereits verwendet wurde.
e. Schreiben einer Markierung in den RFID-Transponder, dass die Fixiervorrichtung bereits verwendet wurde oder Erhöhung eines Verwendungszählers.
f. Automatische Auswahl wenigstens eines optimalen Scanparameters aus Spannung, Strom, Blende, Spirallänge, Wasserkorrekturparameter, Streustrahlenkorrekturparameter, Ringartefaktkorrekturparameter aufgrund der aus dem RFID-Transponder ausgelesenen Daten;
g. Durchführen des Scans, d. h. der Röntgenabbildung.
